# EUROPEAN PATENT APPLICATION

(11) **EP 3 777 824 A1**
(43) Date of publication of application: **17.02.2021**
(21) Application number: 19777768.3
(22) Date of filing: 18.03.2019
(51) Int. Cl.: A61K 8/63, A61K 31/56, A61K 31/704, A61P 17/00, A61P 17/16, A61Q 15/00

(54) **ANTIPERSPIRANT**

(30) Priority: 28.03.2018 JP 2018062833
(71) Applicant: Mandom Corporation, Osaka-shi, Osaka 540-8530 (JP); Osaka University, Suita-shi, Osaka 565-0871 (JP)
(72) Inventor: NAKASHIMA Kie, Suita-shi, Osaka 565-0871 (JP); KURATA Ryuichiro, Osaka-shi, Osaka 540-8530 (JP); FUJITA Fumitaka, Osaka-shi, Osaka 540-8530 (JP); OKADA Fumihiro, Osaka-shi, Osaka 540-8530 (JP)
(74) Representative: TBK
(86) International application number: PCT/JP2019/011137
(87) International publication number: WO 2019/188486

(57) **Abstract**

Provided is an antiperspirant which serves to control secretion of sweat secreted from sweat glands and is capable of doing so by acting on sweat glands without closing sweat pores, the antiperspirant containing, as an active ingredient to control secretion of sweat secreted from sweat glands, at least one type of pentacyclic compound selected from the group consisting of compounds represented by formula (I): 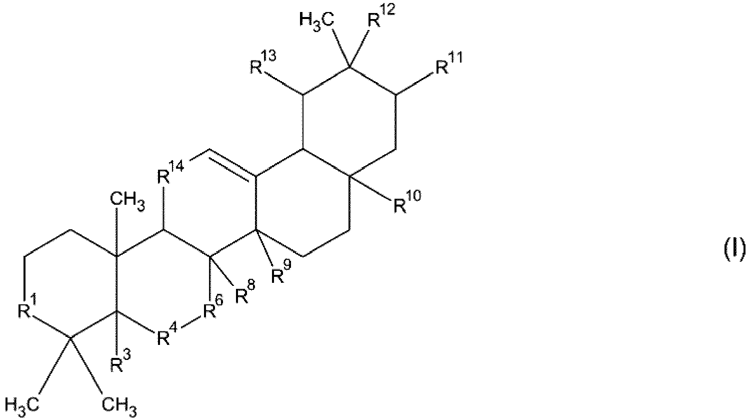 and derivatives thereof, and compounds represented by formula (VII): 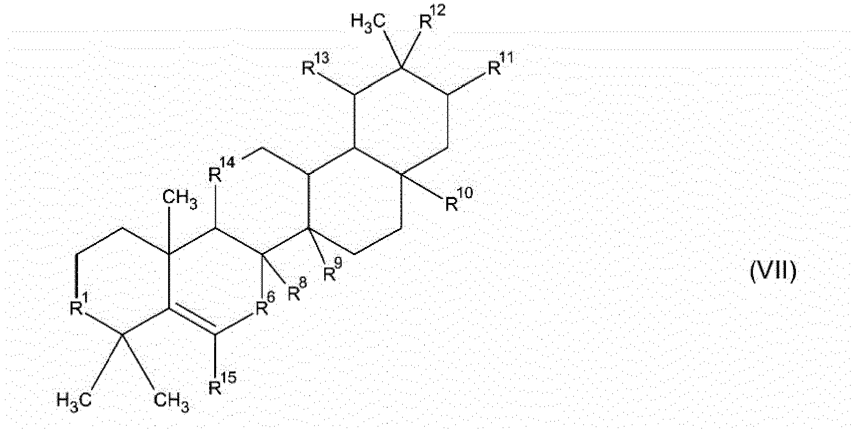 and derivatives thereof.

## Description

### TECHNICAL FIELD

The present invention relates to an antiperspirant. More specifically, the present invention relates to an antiperspirant which is useful as an antiperspirant used in cosmetic uses, an antiperspirant which is used in medical uses, and the like.

### BACKGROUND ART

A human sweat gland has been considered to involve thermoregulation and excretion of waste products when the human sweat gland secretes sweat. When sweat excessively secreted, a skin might become sticky. Therefore, an antiperspirant which blocks a sweat pore of a sweat gland to physically inhibit secretion of sweat has been developed (for example, see Patent Literature 1).

However, when the sweat pore of the sweat gland is blocked, the secretion of sweat might be unnecessarily inhibited. Due to increase in safety consciousness of consumers in recent years, it has been desired to develop a novel antiperspirant which acts on a sweat gland, to control the secretion of sweat without blocking of a sweat pore.

### PRIOR ART LITERATURES

### PATENT LITERATURES

Patent Literature 1: JP H05-000814

### SUMMARY OF THE INVENTION

### PROBLEMS TO BE SOLVED BY THE INVENTION

The present invention has been accomplished in view of the above-mentioned prior art. An object of the present invention is to provide an antiperspirant which acts on a sweat gland, to control secretion of sweat without blocking of a sweat pore.

### MEANS FOR SOLVING THE PROBLEMS

The present invention relates to an antiperspirant for controlling secretion of sweat from a sweat gland, including as an active ingredient for controlling secretion of sweat from a sweat gland, at least one pentacyclic compound selected from the group consisting of a compound represented by the formula (I): 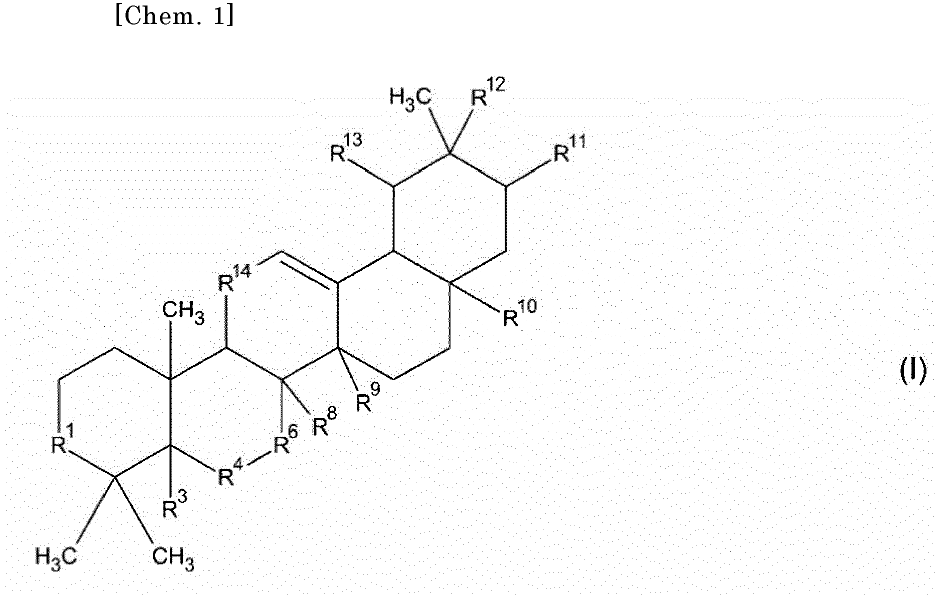 wherein R¹ is a group represented by the formula (II): 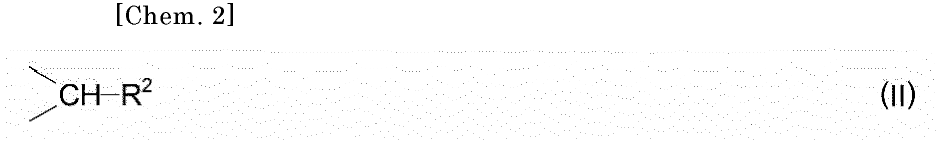 wherein R² is hydrogen atom, hydroxyl group, an alkoxy group having 1 to 12 carbon atoms which may have a substituent, an alkanoyl group having 1 to 12 carbon atoms which may have a substituent, an alkanoyloxy group having 1 to 12 carbon atoms which may have a substituent or glucopyranosyloxy group which may have a substituent, or
a group represented by the formula (III):

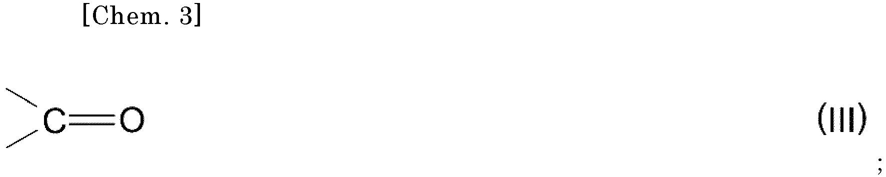

R³ is hydrogen atom or hydroxyl group; R⁴ is the above-mentioned group represented by the formula (III) or a group represented by the formula (IV): 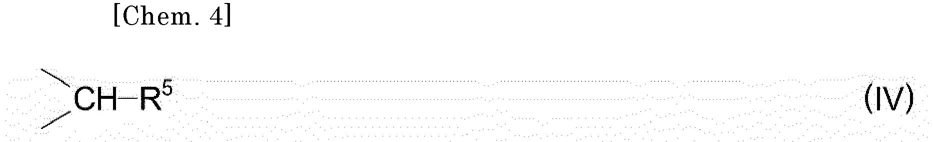 wherein R⁵ is hydrogen atom, a halogen atom, hydroxyl group or an alkyl group having 1 to 6 carbon atoms;
R⁶ is the above-mentioned group represented by the formula (III) or a group represented by the formula (V): 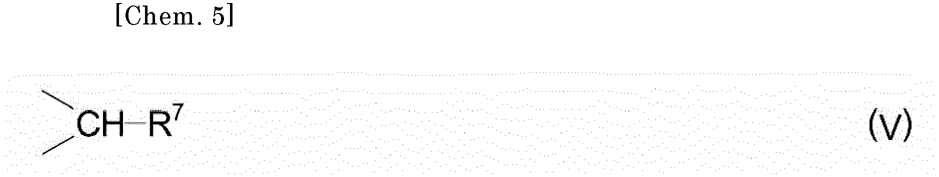 wherein R⁷ is hydrogen atom, hydroxyl group or an alkyl group having 1 to 6 carbon atoms;
each of R⁸ and R⁹ is independently hydrogen atom, hydroxyl group or an alkyl group having 1 to 6 carbon atoms; R¹⁰ is an alkyl group having 1 to 6 carbon atoms or carboxyl group; R¹¹ is hydrogen atom, hydroxyl group, an alkyl group having 1 to 6 carbon atoms or carboxyl group; R¹² is hydrogen atom, an alkyl group having 1 to 6 carbon atoms or carboxyl group; R¹³ is hydrogen atom or an alkyl group having 1 to 6 carbon atoms; and R¹⁴ is the above-mentioned group represented by the formula (III) or a group represented by the formula (VI): 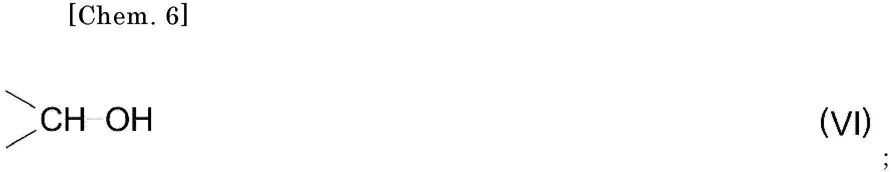 a derivative of the above-mentioned compound represented by the formula (I); a compound represented by the formula (VII): 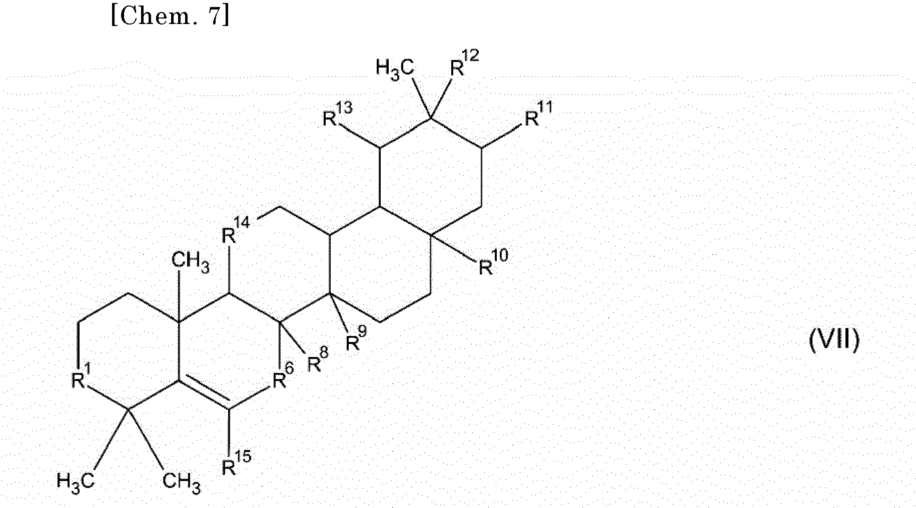 wherein each of R¹, R⁶, R⁸, R⁹, R¹⁰, R¹¹, R¹², R¹³ and R¹⁴ is the same as defined above, and R¹⁵ is hydrogen atom, a halogen atom or hydroxyl group; and
a derivative of the above-mentioned compound represented by the formula (VII).

### EFFECTS OF THE INVENTION

According to the present invention, an antiperspirant which can act on a sweat gland, to control the secretion of sweat without blocking of a sweat pore can be provided.

### BRIEF DESCRIPTION OF THE DRAWINGS

Fig. 1A is a photograph substituted for a drawing, showing a time-lapse photographed image of a part of whole sweat glands after contacting the whole sweat glands with both of the antiperspirant obtained in Example 1 and the sweat gland-stimulating agent obtained in Production Example 3, observed in Test Example 1. Fig. 1B is a photograph substituted for a drawing, showing results of observation of movement of nuclei of cells contained in the region surrounded by a frame shown in Fig. 1A.
Fig. 2A is a graph showing results of changes of the average of moving distances of nuclei of cells of a sweat gland with passage of time after the antiperspirant obtained in Example 1 was contacted with whole sweat glands, examined in Test Example 1. Fig. 2B is a graph showing results of changes of the average of moving distances of nuclei of cells of a sweat gland with passage of time after the control sample used in Comparative Example 1 was contacted with whole sweat glands.
Fig. 3 is a graph showing results of changes of the average of moving distances of nuclei of cells of a secretory duct of a sweat gland with passage of time after the antiperspirant obtained in Example 2 was contacted with whole sweat glands, examined in Test Example 2.
Fig. 4 is a graph showing results of changes of the average of moving distances of nuclei of cells of a secretory duct of a sweat gland with passage of time after the antiperspirant obtained in Example 3 was contacted with whole sweat glands, examined in Test Example 2.
Fig. 5 is a graph showing results of changes of the average of moving distances of nuclei of cells of a secretory duct of a sweat gland with passage of time after the antiperspirant obtained in Example 4 was contacted with whole sweat glands, examined in Test Example 2.
Fig. 6 is a graph showing results of changes of the average of moving distances of nuclei of cells of a secretory duct of a sweat gland with passage of time after the antiperspirant obtained in Example 5 was contacted with whole sweat glands, examined in Test Example 2.
Fig. 7 is a graph showing results of changes of the average of moving distances of nuclei of cells of a secretory duct of a sweat gland with passage of time after the antiperspirant obtained in Example 6 was contacted with whole sweat glands, examined in Test Example 2.
Fig. 8 is a graph showing results of changes of the average of moving distances of nuclei of cells of a secretory duct of a sweat gland with passage of time after the antiperspirant obtained in Example 7 was contacted with whole sweat glands, examined in Test Example 2.

### MODE FOR CARRING OUT THE INVENTION

The antiperspirant of the present invention is an antiperspirant for controlling secretion of sweat, which is characterized in that the antiperspirant includes as an active ingredient for controlling secretion of sweat from a sweat gland, at least one pentacyclic compound selected from the group consisting of a compound represented by the formula (I): 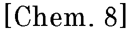 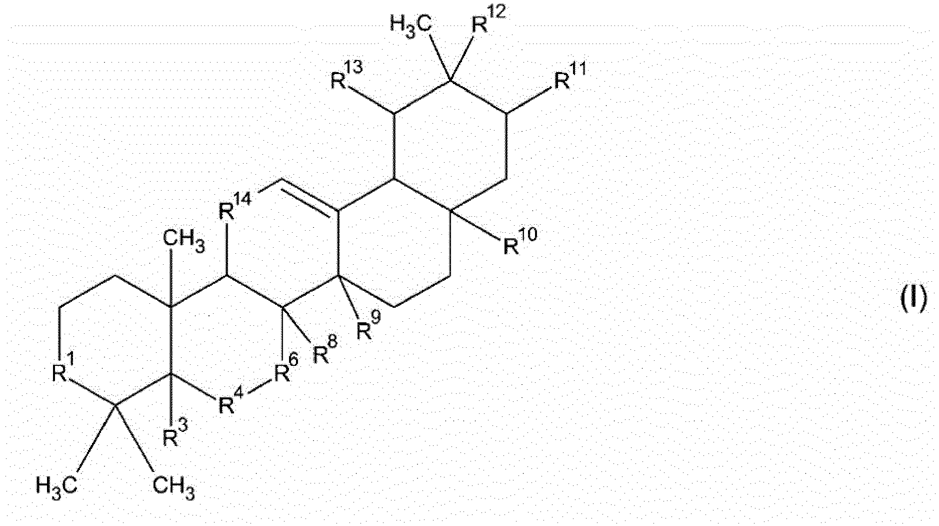 wherein R¹ is a group represented by the formula (II): 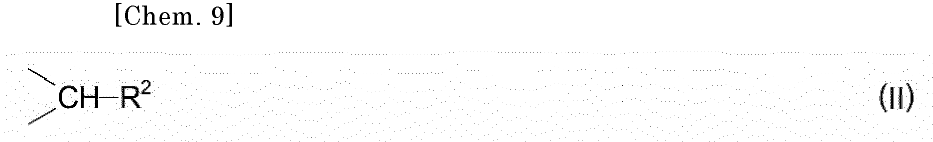 wherein R² is hydrogen atom, hydroxyl group, an alkoxy group having 1 to 12 carbon atoms which may have a substituent, an alkanoyl group having 1 to 12 carbon atoms which may have a substituent, an alkanoyloxy group having 1 to 12 carbon atoms which may have a substituent or glucopyranosyloxy group which may have a substituent, or
a group represented by the formula (III):

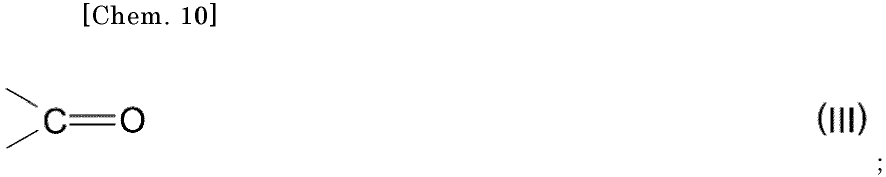

R³ is hydrogen atom or hydroxyl group; R⁴ is the above-mentioned group represented by the formula (III) or a group represented by the formula (IV): 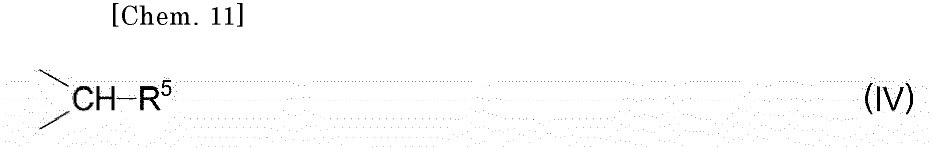 wherein R⁵ is hydrogen atom, a halogen atom, hydroxyl group or an alkyl group having 1 to 6 carbon atoms;
R⁶ is the above-mentioned group represented by the formula (III) or a group represented by the formula (V): 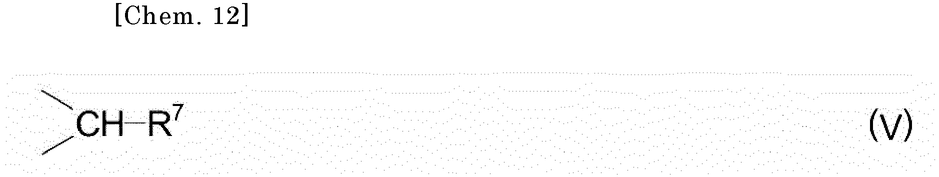 wherein R⁷ is hydrogen atom, hydroxyl group or an alkyl group having 1 to 6 carbon atoms;
each of R⁸ and R⁹ is independently hydrogen atom, hydroxyl group or an alkyl group having 1 to 6 carbon atoms; R¹⁰ is an alkyl group having 1 to 6 carbon atoms or carboxyl group; R¹¹ is hydrogen atom, hydroxyl group, an alkyl group having 1 to 6 carbon atoms or carboxyl group; R¹² is hydrogen atom, an alkyl group having 1 to 6 carbon atoms or carboxyl group; R¹³ is hydrogen atom or an alkyl group having 1 to 6 carbon atoms; and R¹⁴ is the above-mentioned group represented by the formula (III) or a group represented by the formula (VI): 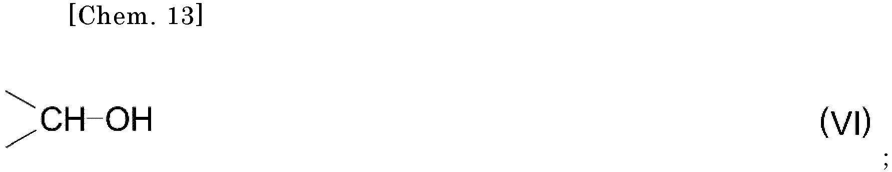 a derivative of the above-mentioned compound represented by the formula (I); a compound represented by the formula (VII): 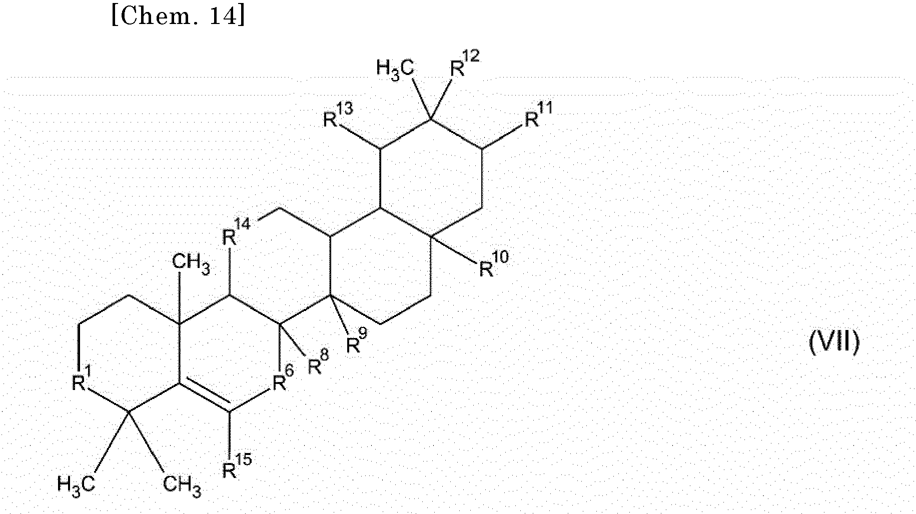 wherein each of R¹, R⁶, R⁸, R⁹, R¹⁰, R¹¹, R¹², R¹³ and R¹⁴ is the same as defined above, and R¹⁵ is hydrogen atom, a halogen atom or hydroxyl group; and
a derivative of the compound represented by the formula (VII).

The compound represented by the formula (I), the derivative of the compound represented by the formula (I), the compound represented by the formula (VII) and the derivative of the compound represented by the formula (VII) act on a secretory duct of a human sweat gland, to control movement of the secretory duct of the sweat gland in secreting sweat. Accordingly, the antiperspirant of the present invention can act on the sweat gland, to control the secretion of sweat without blocking of the sweat pore.

In the compound represented by the formula (I), R¹ is the group represented by the formula (II) or the group represented by the formula (III).

In the group represented by the formula (II), R² is hydrogen atom, hydroxyl group, an alkoxy group having 1 to 12 carbon atoms which may have a substituent, an alkanoyl group having 1 to 12 carbon atoms which may have a substituent, an alkanoyloxy group having 1 to 12 carbon atoms which may have a substituent, or glucopyranosyloxy group which may have a substituent.

The alkoxy group having 1 to 12 carbon atoms includes, for example, methoxy group, ethoxy group, n-propoxy group, cyclopropoxy group, n-butoxy group, isobutoxy group, tert-butoxy group, cyclobutoxy group, n-pentyloxy group, n-hexyloxy group, n-heptyloxy group, n-octyloxy group, n-nonyloxy group, n-decyloxy group, n-dodecyloxy group, 2-ethylhexyloxy group, cyclohexyloxy group, cyclooctyloxy group and the like, and the present invention is not limited only to those exemplified ones. The substituent which the alkoxy group having 1 to 12 carbon atoms may have includes, for example, a halogen atom; an alkyl group having 1 to 6 carbon atoms such as methyl group, ethyl group, n-propyl group, isopropyl group, n-butyl group, isobutyl group or tert-butyl group; hydroxyl group; amino group; carboxyl group; cyano group; nitro group; thiol group; and the like, and the present invention is not limited only to those exemplified ones.

The alkanoyl group having 1 to 12 carbon atoms includes, for example, formyl group, acetyl group, n-propionyl group, n-butyryl group, isobutyryl group, n-pentanoyl group, tert-butylcarbonyl group, n-hexanoyl group and the like, and the present invention is not limited only to those exemplified ones. The substituent which the alkanoyl group having 1 to 12 carbon atoms may have includes, for example, a halogen atom, an alkyl group having 1 to 6 carbon atoms, hydroxyl group, amino group, carboxyl group, cyano group, nitro group, thiol group and the like, and the present invention is not limited only to those exemplified ones. The alkyl group having 1 to 6 carbon atoms used as the substituent which the alkanoyl group may have can be the same as the alkyl group having 1 to 6 carbon atoms used in the substituent which the alkoxy group having 1 to 12 carbon atoms may have.

The alkanoyloxy group having 1 to 12 carbon atoms includes, for example, formyloxy group, acetyloxy group, n-propionyloxy group, n-butyryloxy group, isobutyryloxy group, n-pentanoyloxy group, tert-butylcarbonyloxy group, n-hexanoyloxy group and the like, and the present invention is not limited only to those exemplified ones. The substituent which the alkanoyloxy group having 1 to 12 carbon atoms may have includes, for example, a halogen atom, an alkyl group having 1 to 6 carbon atoms, hydroxyl group, amino group, carboxyl group, cyano group, nitro group, thiol group, a heterocyclic group which may have a substituent and the like, and the present invention is not limited only to those exemplified ones. The alkyl group having 1 to 6 carbon atoms used as the substituent which the alkanoyloxy group may have can be the same as the alkyl group having 1 to 6 carbon atoms which the alkoxy group having 1 to 12 carbon atoms may have. The heterocyclic group includes, for example, furyl group, pyridyl group, thienyl group, quinolyl group, isoquinolyl group, imidazolyl group, oxazolyl group, thiazolyl group, benzoxazolyl group, benzoisoxazolyl group, benzothiazolyl group, benzisothiazolyl group and the like, and the present invention is not limited only to those exemplified ones. The substituent which the heterocyclic group may have includes, for example, an alkyl group having 1 to 6 carbon atoms, hydroxyl group, nitro group, cyano group and the like, and the present invention is not limited only to those exemplified ones. The alkyl group having 1 to 6 carbon atoms used as the substituent which the heterocyclic group may have can be the same as the alkyl group having 1 to 6 carbon atoms used in the substituent which the alkoxy group having 1 to 12 carbon atoms may have.

The substituent which the glucopyranosyloxy group may have includes, for example, an alkyl group having 1 to 6 carbon atoms, hydroxyl group, nitro group, cyano group, glucopyranolofuranosyl group and the like, and the present invention is not limited only to those exemplified ones. The alkyl group having 1 to 6 carbon atoms used as the substituent which the glucopyranosyloxy group may have can be the same as the alkyl group having 1 to 6 carbon atoms used in the substituent which the alkoxy group having 1 to 12 carbon atoms may have.

In the group of R¹, the group represented by the formula (II) is preferable from the viewpoint of improvement in antiperspirant efficacy. In this case, R² included in the formula (II) is preferably hydroxy group, an alkanoyloxy group having 1 to 12 carbon atoms which may have a substituent or glucopyranolonosyloxy group which may have a substituent, and more preferably hydroxyl group, an alkanoyloxy group of 1 to 12 carbon atoms having carboxyl group as a substituent or a glucopyranolonosyloxy group having glucopyranolofuranosyl group as a substituent, from the viewpoint of improvement in antiperspirant efficacy.

The group of R³ is hydrogen atom or hydroxyl group. In the group of R³, hydrogen atom is preferable from the viewpoint of improvement in antiperspirant efficacy.

The group of R⁴ is the group represented by the formula (III) or the group represented by the formula (IV). In the group represented by the formula (IV), R⁵ is hydrogen atom, a halogen atom, hydroxyl group or an alkyl group having 1 to 6 carbon atoms. The alkyl group having 1 to 6 carbon atoms used in R⁵ can be the same as the alkyl group having 1 to 6 carbon atoms used in the substituent which the alkoxy group having 1 to 12 carbon atoms may have. In the group of R⁴, the group represented by the formula (IV) is preferable from the viewpoint of improvement in antiperspirant efficacy. In this case, R⁵ included in the formula (IV) is preferably hydrogen atom.

R⁶ is the group represented by the formula (III) or the group represented by the formula (V). In the group represented by the formula (V), R⁷ is hydrogen atom, hydroxyl group or an alkyl group having 1 to 6 carbon atoms. The alkyl group having 1 to 6 carbon atoms used in R⁷ can be the same as the alkyl group having 1 to 6 carbon atoms used in the substituent which the alkoxy group having 1 to 12 carbon atoms may have. R⁶ is preferably the group represented by the formula (V) from the viewpoint of improvement in antiperspirant efficacy. In this case, R⁷ included in the formula (V) is preferably hydrogen atom.

Each of R⁸ and R⁹ is independently hydrogen atom, hydroxyl group or an alkyl group having 1 to 6 carbon atoms. The alkyl group having 1 to 6 carbon atoms used in R⁸ and R⁹ can be the same as the alkyl group having 1 to 6 carbon atoms used in the substituent which the alkoxy group having 1 to 12 carbon atoms may have. R⁸ is preferably hydrogen atom or an alkyl group having 1 to 6 carbon atoms, and more preferably an alkyl group having 1 to 6 carbon atoms, from the viewpoint of improvement in antiperspirant efficacy. R⁹ is preferably hydrogen atom or an alkyl group having 1 to 6 carbon atoms, and more preferably an alkyl group having 1 to 6 carbon atoms, from the viewpoint of improvement in antiperspirant efficacy.

R¹⁰ is an alkyl group having 1 to 6 carbon atoms or carboxyl group. The alkyl group having 1 to 6 carbon atoms used in R¹⁰ can be the same as the alkyl group having 1 to 6 carbon atoms used in the substituent which the alkoxy group having 1 to 12 carbon atoms may have. R¹⁰ is preferably an alkyl group having 1 to 6 carbon atoms, more preferably ethyl group, from the viewpoint of improvement in antiperspirant efficacy.

R¹¹ is hydrogen atom, hydroxyl group, an alkyl group having 1 to 6 carbon atoms or carboxyl group. The alkyl group having 1 to 6 carbon atoms used in R¹¹ can be the same as the alkyl group having 1 to 6 carbon atoms used in the substituent which the alkoxy group having 1 to 12 carbon atoms may have. R¹¹ is preferably hydrogen atom or hydroxyl group, more preferably hydrogen atom, from the viewpoint of improvement in antiperspirant efficacy.

R¹² is hydrogen atom, an alkyl group having 1 to 6 carbon atoms or carboxyl group. The alkyl group having 1 to 6 carbon atoms used in R¹² can be the same as the alkyl group having 1 to 6 carbon atoms used in the substituent which the alkoxy group having 1 to 12 carbon atoms may have. R¹² is preferably carboxyl group from the viewpoint of improvement in antiperspirant efficacy.

R¹³ is hydrogen atom or an alkyl group having 1 to 6 carbon atoms. The alkyl group having 1 to 6 carbon atoms used in R¹³ can be the same as the alkyl group having 1 to 6 carbon atoms used in the substituent which the alkoxy group having 1 to 12 carbon atoms may have. R¹³ is preferably hydrogen atom from the viewpoint of improvement in antiperspirant efficacy.

R¹⁴ is the group represented by the formula (III) or the group represented by the formula (VI). R¹⁴ is preferably the group represented by the formula (III) from the viewpoint of improvement in antiperspirant efficacy.

The derivative of the compound represented by the formula (I) includes, for example, a metal salt of the compound represented by the formula (I), an inorganic base salt of the compound represented by the formula (I), an organic amine salt of the compound represented by the formula (I), an ester of the compound represented by the formula (I) and the like, and the present invention is not limited only to those exemplified ones. The metal salt includes, for example, an alkali metal salt such as a sodium salt or a potassium salt; an alkaline earth metal salt such as a magnesium salt or a calcium salt; and the like, and the present invention is not limited only to those exemplified ones. The inorganic base salt includes, for example, an ammonium salt and the like, and the present invention is not limited only to those exemplified ones. The organic amine salt includes a triethanolamine salt, a triethylamine salt and the like, and the present invention is not limited only to those exemplified ones. The ester of the compound represented by the formula (I) includes, for example, an ester made of the compound represented by the formula (I) and an alcohol having 1 to 24 carbon atoms, an ester made of the compound represented by the formula (I) and pyridoxine, and the like, and the present invention is not limited only to those exemplified ones. The alcohol having 1 to 24 carbon atoms includes, for example, lower alcohols such as methyl alcohol, butyl alcohol and propyl alcohol; higher alcohols such as myristyl alcohol, cetyl alcohol, stearyl alcohol, behenyl alcohol and oleyl alcohol; polyhydric alcohols such as glycerol; and the like, and the present invention is not limited only to those exemplified ones.

As the compound represented by the formula (I) and the derivative thereof, commercially available compounds can be used. The commercially available compounds include, for example, carbenoxolone, carbenoxolone disodium, glycyrrhetinic acid, glycyrrhizic acid, INI-0602, ursolic acid, oleanolic acid, oleanonic acid, α-amylin, β-amylin and the like, and the present invention is not limited only to those exemplified ones. The compound represented by the formula (I) and the derivative thereof can be easily prepared by synthesis. The glycyrrhetinic acid can be 18α-glycyrrhetinic acid. Alternatively, the glycyrrhetinic acid can be 18β-glycyrrhetinic acid.

In the compound represented by the formula (VII), R¹, R⁶, R⁸, R⁹, R¹⁰, R¹¹, R¹², R¹³ and R¹⁴ are the same as R¹, R⁶, R⁸, R⁹, R¹⁰, R¹¹, R¹², R¹³, and R¹⁴ included in the compound represented by the formula (I). R¹⁵ is hydrogen atom, a halogen atom or hydroxyl group.

The derivative of the compound represented by the formula (VII) includes, for example, a metal salt of the compound represented by the formula (VII), an inorganic base salt of the compound represented by the formula (VII), an organic amine salt of the compound represented by the formula (VII), an ester of the compound represented by the formula (VII) and the like, and the present invention is not limited only to those exemplified ones. The metal salt include, for example, an alkali metal salt such as a sodium salt or a potassium salt; an alkaline earth metal salt such as a magnesium salt or a calcium salt; and the like, and the present invention is not limited only to those exemplified ones. The inorganic base salt includes, for example, an ammonium salt and the like, and the present invention is not limited only to those exemplified ones. The organic amine salt includes, for example, a triethanolamine salt, a triethylamine salt and the like, and the present invention is not limited only to those exemplified ones. The ester of the compound represented by the formula (VII) includes, for example, an ester made of the compound represented by the formula (VII) and an alcohol having 1 to 24 carbon atoms, an ester made of the compound represented by the formula (VII) and pyridoxine, and the like, and the present invention is not limited only to those exemplified ones. The alcohol having 1 to 24 carbon atoms includes, for example, lower alcohols such as methyl alcohol, butyl alcohol and propyl alcohol; higher alcohols such as myristyl alcohol, cetyl alcohol, stearyl alcohol, behenyl alcohol and oleyl alcohol; and polyhydric alcohols such as glycerol; and the like, and the present invention is not limited only to those exemplified ones.

As the compound represented by the formula (VII) and the derivative thereof, commercially available compounds can be used. The compound represented by the formula (VII) and the derivative thereof can be easily prepared by synthesis.

The pentacyclic compounds can be used alone or in combination of at least two kinds thereof. Among the pentacyclic compounds, the compound represented by the formula (I) and the derivative thereof are preferable; the compound represented by the formula (I) in which R¹ is the group represented by the formula (II), R³ is hydrogen atom, R⁴ is the group represented by the formula (IV), R⁶ is the group represented by the formula (V), each of R⁸ and R⁹ is an alkyl group having 1 to 6 carbon atoms respectively, R¹⁰ is methyl group, R¹¹ is hydrogen atom or hydroxyl group, R¹² is carboxyl group, R¹³ is hydrogen atom, and R¹⁴ is the group represented by the formula (III), and the derivative thereof are more preferable; a compound represented by the formula (VIII): 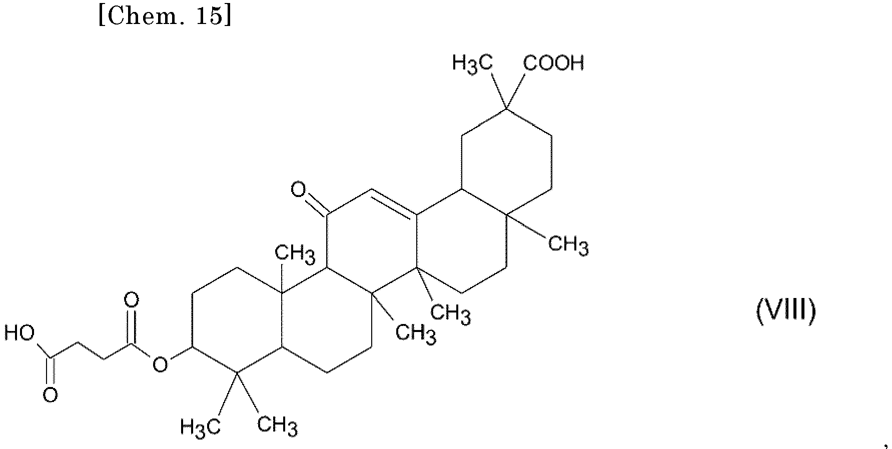 an alkali metal salt of the compound represented by the formula (VIII), a compound represented by the formula (IX): 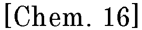 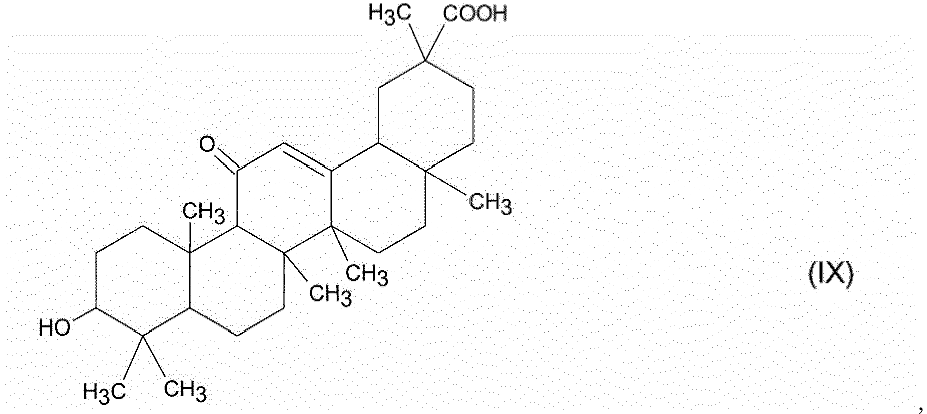 an alkali metal salt of the compound represented by the formula (IX), an ester made of the compound represented by the formula (IX) and an alcohol having 1 to 24 carbon atoms, an ester made of the compound represented by the formula (IX) and pyridoxine, a compound represented by the formula (X): 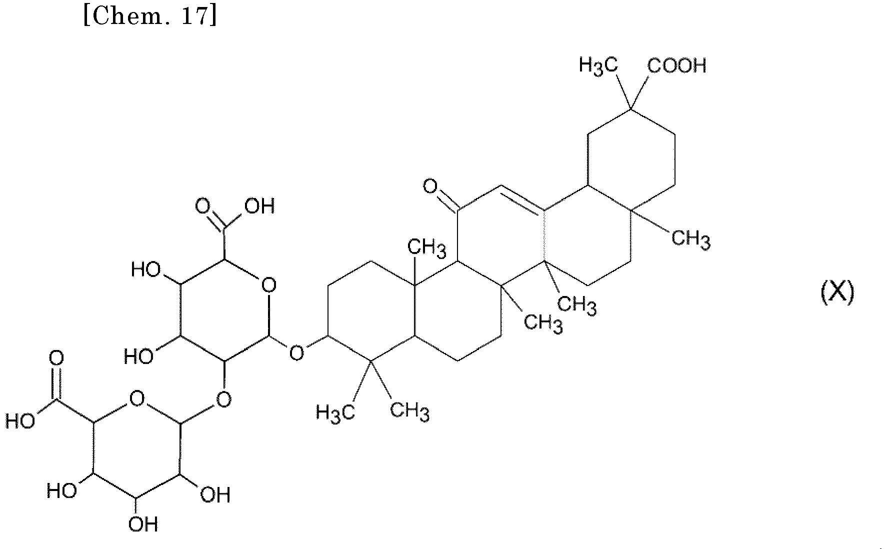 an alkali metal salt of the compound represented by the formula (X) and an ammonium salt of the compound represented by the formula (X) are furthermore preferable; and the compound represented by the formula (VIII) and an alkali metal salt of the compound represented by the formula (VIII) are even more preferable, from the viewpoint of improvement in antiperspirant action.

The content of the pentacyclic compound in the antiperspirant of the present invention is preferably 0.0000001% by mass or more, more preferably 0.0001% by mass or more, from the viewpoint of sufficient expression of antiperspirant efficacy. The content of the pentacyclic compound in the antiperspirant is preferably 10% by mass or less, and more preferably 0.6% by mass or less, from the viewpoint of reduction of burden imposed on users.

The antiperspirant of the present invention can be formulated with an auxiliary agent within a scope which would not hinder an object of the present invention. The auxiliary agent includes, for example, a surfactant, an alcohol, a refreshing agent, a plant extract, a perfume, a metal-sequestering agent, an antioxidant, an antiseptic, a thickener and the like, and the present invention is not limited only to those exemplified ones.

As explained above, since the antiperspirant of the present invention includes the above-mentioned pentacyclic compound as an active ingredient for controlling the secretion of sweat secreted from the sweat gland, the antiperspirant can act on a secretory duct of the sweat gland, to control movement of the secretory duct of the sweat gland in secretion of sweat. Thus, the antiperspirant of the present invention can act on the sweat gland, to control the secretion of sweat without blocking of a sweat pore. Accordingly, it is expected that the antiperspirant of the present invention is used as, for example, an antiperspirant used in cosmetic uses, an antiperspirant used in medical uses and the like. In addition, it is expected that the antiperspirant of the present invention is used as an antiperspirant in response to a user's demand, such as an antiperspirant for completely inhibiting the secretion of sweat or an antiperspirant for alleviating the secretion of sweat.

### EXAMPLES

The present invention will be more specifically explained based on the following working examples. However, the present invention is not limited only to the working examples. Incidentally, meanings of abbreviations used in the working examples and the like are as follows:

### <Explanation of abbreviations>

HEPES: 2-[4-(2-hydroxyethyl) piperazin-1-yl] ethanesulfonic acid
PBS: phosphate-buffered saline
5×PBS: 5-fold concentration of phosphate-buffered saline

### Production Example 1

To 500 pL of PBS, 3.5 pL of a cytoskeleton-staining reagent manufactured by Cytoskeleton under the trade name of Acti-stain 488 (concentration of Acti-stain 488: 14 pM), 4 pL of a cell membrane-staining reagent manufactured by Thermo Fisher Scientific under the trade name of CellMask and 1 pL of a nuclear staining reagent (Hoechst 33342) were added, to obtain a staining reagent mixture.

### Production Example 2

A collagen-containing solution was prepared by mixing 700 pL of collagen type I-A solution (content of collagen type I-A: 3% by mass) manufactured by Nitta Gelatin Inc. with 200 pL of 5×PBS [composition: 685 mM sodium chloride, 13.5 mM potassium chloride, 50 mM disodium hydrogenphosphate dodecahydrate and 9 mM potassium dihydrogenphosphate, pH 7.4] and 100 pL of a collagen reconstitution buffer [composition: 50 mM sodium hydroxide, 260 mM HEPES and 200 mM sodium bicarbonate, pH 10.0].

### Production Example 3

A sweat gland-stimulating agent was prepared by adding pyrocarpin as a sweat gland contraction-inducing reagent to PBS so that the concentration of the pyrocarpin was 100 mM.

### Example 1

An antiperspirant was prepared by adding the compound represented by the formula (VIII) to PBS so that the concentration of the compound was 100 mM.

### Comparative Example 1

In the followings, PBS was used as a control sample of Comparative Example 1.

### Test Example 1

### (1) Isolation of Whole Sweat Glands

As skin tissues, skin tissues which were refrigerated immediately after isolating from living donors and stored at 4°C for 15 hours were used. The skin tissues were immersed in PBS containing 10µM staining agent (Neutral Red), to label the sweat glands included in the skin tissues. Next, the dermis of each of the skin tissues including the staining agent was finely cut to obtain dermal segments. Whole sweat glands were collected from the dermal segments by using tweezers under an optical microscope.

### (2) Staining

The whole sweat glands obtained in Test Example 1(1) were immersed in the staining reagent mixture obtained in Production Example 1 at room temperature (24°C) for 30 minutes, to stain the whole sweat glands. The stained whole sweat glands were washed with PBS.

### (3) Positional anchorage of stained whole sweat glands on a support

The washed whole sweat glands obtained in Test Example 1(2) were allowed to stand on a glass bottom dish under a stereoscopic microscope manufactured by Leica under the trade name of M125. The collagen-containing liquid obtained in Production Example 2 was added dropwise to the whole sweat glands that were allowed to stand on the glass bottom dish. Next, the above-mentioned glass bottom dish was incubated at 37°C for 5 minutes to gelate the collagen type I-A contained in the collagen-containing liquid placed on the whole sweat glands. Thereafter, 300 µL of PBS was added onto the above-mentioned whole sweat glands so as not to dry the whole sweat glands. Thus, the whole sweat glands were held on the glass bottom dish used as a support with the collagen type I-A so that the position of each of the whole sweat glands was retained, to obtain an observation sample.

### (4) Observation

While adhesion of the whole sweat glands to the glass bottom dish with the gelated collagen type I-A was confirmed under the stereoscopic microscope, an aliquot of Dulbecco's phosphate buffer [manufactured by Gibco, Catalog No. 14190-144] was added to the glass bottom dish used as a support.

The antiperspirant obtained in Example 1 in an amount of 3 µL was contacted with the whole sweat glands on the glass bottom dish in the observation sample. The observation of the whole sweat glands on the glass bottom dish with the time-lapse imaging was initiated by using a confocal laser scanning microscope for biological uses [inverted microscope manufactured by Olympus Corporation under the trade name of IX-83 equipped with a device manufactured by Olympus Corporation under the trade name of FV 1200]. At that time, the whole sweat glands were visualized by detecting fluorescence derived from the nuclear staining reagent bound to the nuclei and fluorescence derived from the cytoskeleton-staining reagent bound to the cytoskeleton. After 600 seconds passed from the initiation of the observation, 30 µL of the sweat gland-stimulating agent obtained in Production Example 3 was contacted with the whole sweat glands on the glass bottom dish, and thereafter the observation with the time-lapse imaging was continued. The conditions of time-lapse imaging are as follows:

### <Conditions of time-lapse imaging>

X: 634.662 µm
Y: 634.662 µm
Z: 30 µm
Z interval :1.5 µm
Number of Z sheets: 21 sheets
Time interval: 30 seconds
Total number of frames: 61 frames
Total observation time: 1800 seconds

Next, five cells were selected from a cell group in the lumen of the secretory duct in the time-lapse photographed image of the whole sweat glands, and five cells were selected from a cell group in the outer periphery of the secretory duct. Thereafter, changes of moving distances of nuclei of each cell with passage of time were examined by using an image analysis software manufactured by National Institutes of Health (NIH) under the trade name of ImageJ. In order to measure a moving distance of the nuclei of the cell, firstly changes (differences) in coordinates between consecutive frames were calculated by using X-coordinate and Y-coordinate of the nuclei of each cell in accordance with the formulae (XI) and (XII):

Xₙ₊₁-Xₙ (XI)

wherein n is an integer of 1 to 61,

Yₙ₊₁-Yₙ (XII)

wherein n is the same as mentioned above. Next, in accordance with the Pythagorean theorem (a²+b²=c²), a square root of the formula (XIII):

(Xₙ₊₁-Xₙ)² + (Yₙ₊₁-Yn)² (XIII)

wherein n is the same as mentioned above,
was calculated, to obtain a movement value between the nuclei of the cells in each successive frame. Furthermore, in order to determine an actual moving distance, a length of a pixel (µm/pixel) was obtained from the area of the obtained image [634.662 µm × 634.662 µm] and the pixel (512 × 512). The actual moving distance (µm) of the nuclei of the cell was calculated by multiplying the movement value by the length of a pixel. Next, the average of moving distances of the nuclei of the cells in the lumen of the secretory duct and the average of moving distances of the nuclei of the cells in the outer periphery of the secretory duct were examined by using the moving distance of the nuclei of each cell.

In addition, the average of the moving distances of the nuclei of the cells in the lumen of the secretory duct and the average of the moving distances of the nuclei of the cells in the outer periphery of the secretory duct were examined in the same manner as in the above except that the control sample of Comparative Example 1 was used in place of the antiperspirant obtained in Example 1.

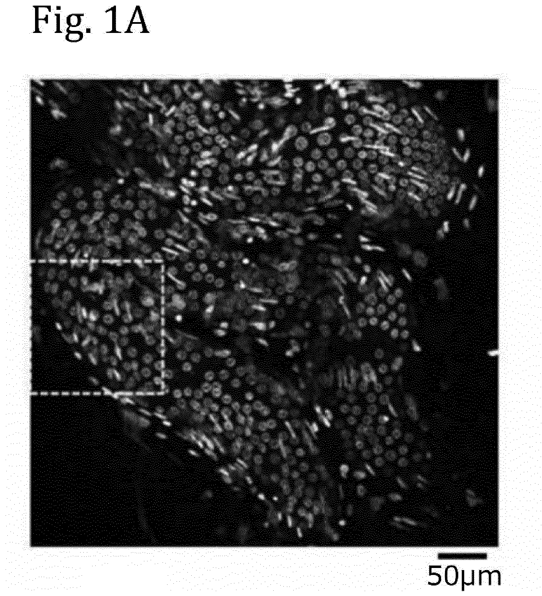

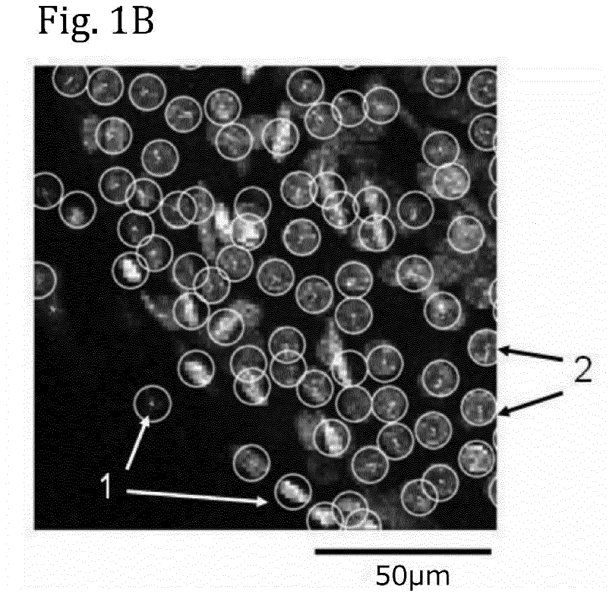

In Test Example 1, a time-lapse photographed image of a part of the whole sweat glands after contacting the whole sweat glands with both of the antiperspirant obtained in Example 1 and the sweat gland-stimulating agent obtained in Production Example 3 is shown in Fig. 1A, and results of the observation of movement of the nuclei of the cells included in the region surrounded by a frame are shown in Fig. 1B. The scale bar shown in Fig. 1A means 50 µm, and the scale bar shown in Fig. 1B means 50 µm. In the figures, 1 denotes a representative example of a muscle epithelial cell, and 2 denotes a representative example of a lumen cell.

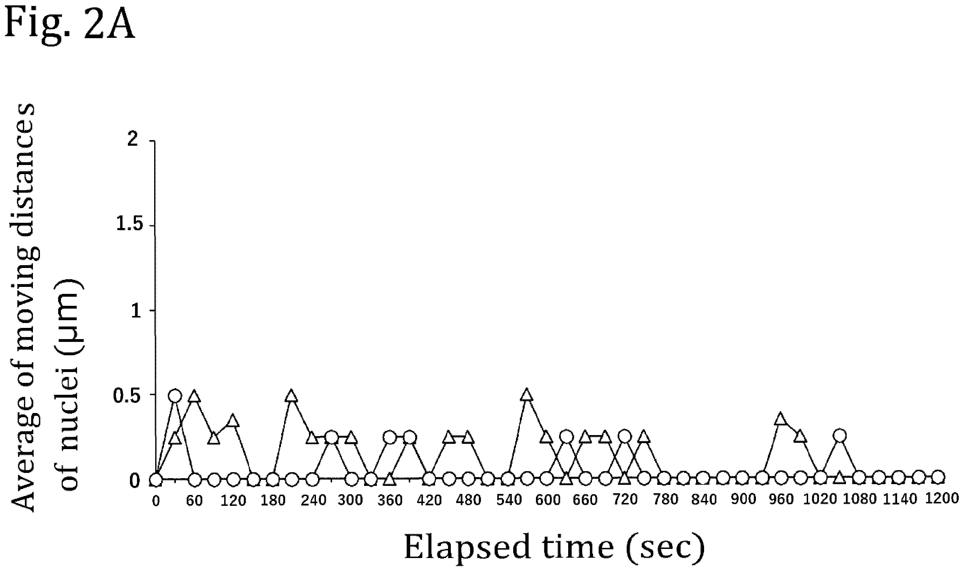

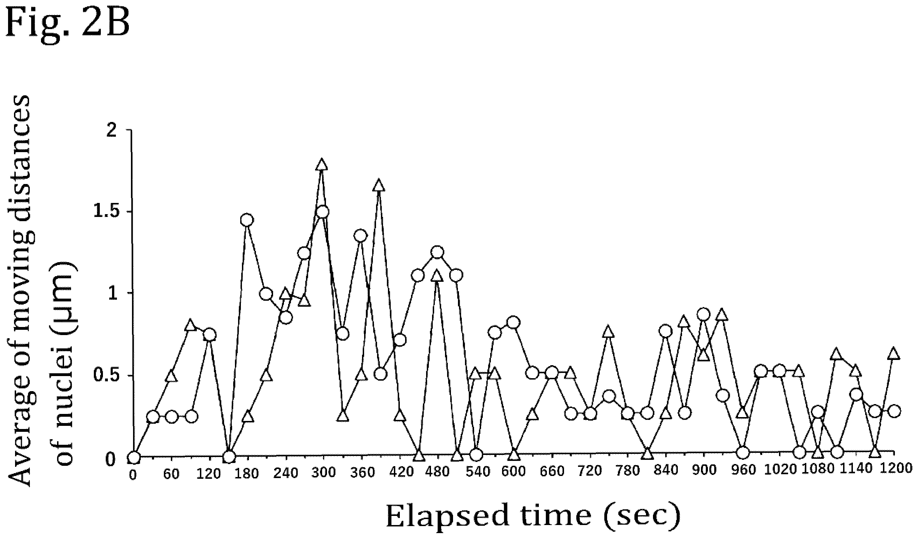

In addition, in Test Example 1, results for examining changes of the average of moving distances of nuclei of cells of a sweat gland with passage of time after the antiperspirant obtained in Example 1 was contacted with whole sweat glands are shown in Fig. 2A, and results for examining changes of the average of moving distances of nuclei of cells of the sweat gland with passage of time after the control sample of Comparative Example 1 was contacted with the whole sweat glands are shown in Fig. 2B. In the figures, a white triangle means changes of the average of moving distances of nuclei of cells in the outer periphery of a secretory duct of the sweat gland with passage of time, and a white circle means changes of the average of moving distances of nuclei of cells included in the lumen of a secretory duct of the sweat gland with passage of time. In addition, in the figures, the "Average of moving distances of nuclei" means an average of moving distances of nuclei of cells of a secretory duct of the sweat gland.

From the results shown in Fig. 1 and Fig. 2A, it can be seen that the nuclei of the cells of the lumen of the secretory duct of the sweat gland [shown as white circle in Fig. 2A] and the nuclei of the cells of the outer periphery of the secretory duct of the sweat gland [shown as white triangle in Fig. 2A] are hardly moved even after the sweat gland-stimulating agent obtained in Production Example 3 was contacted with the whole sweat glands when the antiperspirant obtained in Example 1 was previously contacted with the whole sweat glands.

On the contrary, it can be seen from the results shown in Fig. 2B that both of the nuclei of the cells of the lumen of the secretory duct of the sweat gland [shown as white circle in Fig. 2B] and the nuclei of the cells of the outer periphery of the secretory duct of the sweat gland [shown as white triangle in Fig. 2B] moved after contacting the sweat gland-stimulating agent obtained in the Production Example 3 with the whole sweat glands when the control sample of Comparative Example 1 was contacted with the whole sweat glands.

In addition, it was confirmed from the results for observing the secretory duct of the sweat gland in the time-lapse photographed image that movement of the secretory duct of the sweat gland was suppressed by contacting the antiperspirant obtained in Example 1 with the whole sweat glands.

It can be seen from these results that the antiperspirant obtained in Example 1 acts on the secretory duct of the sweat gland, and suppresses movement of cells of the secretory duct, and thereby movement of the secretory duct is suppressed. Thus, it can be seen that the antiperspirant obtained in Example 1 controls movement of the sweat gland, and thereby the secretion of sweat is controlled.

Incidentally, when an antiperspirant containing the compound represented by the formula (IX), the compound represented by the formula (X), the compound represented by the formula (I) other than the compounds represented by the formulae (IX) and (X), a derivative of the compound represented by the formula (I), the compound represented by the formula (VII) or a derivative of the compound represented by the formula (VII) is used in place of the antiperspirant obtained in Example 1, it is recognized that each of the antiperspirants shows the same tendency as the antiperspirant obtained in Example 1.

From the above results, the antiperspirant of the present invention can act on the secretory duct of the sweat gland, to control movement of the secretory duct of the sweat gland in secreting sweat. Accordingly, it can be seen that the antiperspirant of the present invention controls movement of the sweat gland, to control the secretion of sweat without blocking of sweat pores.

### Example 2

To PBS was added 18a-glycyrrhetinic acid so that the concentration of the 18α-glycyrrhetinic acid was 10 mM, to obtain an antiperspirant.

### Example 3

To PBS was added 18β-glycyrrhetinic acid so that the concentration of the 18β-glycyrrhetinic acid was 10 mM, to obtain an antiperspirant.

### Example 4

To PBS was added ursolic acid so that the concentration of the ursolic acid was 10 mM, to obtain an antiperspirant.

### Example 5

To PBS was added oleanolic acid so that the concentration of the oleanolic acid was 100 µM, to obtain an antiperspirant.

### Example 6

To PBS was added α-amylin so that the concentration of the α-amylin was 2 mM, to obtain an antiperspirant.

### Example 7

To PBS was added β-amylin so that the concentration of the β-amylin was 2 mM, to obtain an antiperspirant.

### Production Example 4

To PBS was added pyrocalpin so that the concentration of the pyrocalpin was 10 mM, to obtain an antiperspirant.

### Test Example 2

The same procedures as in Test Example 1 were carried out, except that each of the antiperspirants obtained in Examples 2 to 7 was used in place of the antiperspirant obtained in Example 1, that the sweat gland-stimulating agent obtained in the Production Example 4 was used in place of the sweat gland-stimulating agent obtained in the Production Example 3, and that five cells were selected from a cell group of the secretory duct in the time-lapse photographed image of the whole sweat glands in place of selecting of a cell group of the lumen of the secretory duct in the time-lapse photographed image of the whole sweat glands and a cell group of the outer periphery of the secretory duct. Each of the antiperspirants obtained in Examples 2 to 7 was contacted with the whole sweat glands respectively, and change of the average of moving distances of nuclei of cells of a secretory duct of the sweat gland was examined with passage of time.

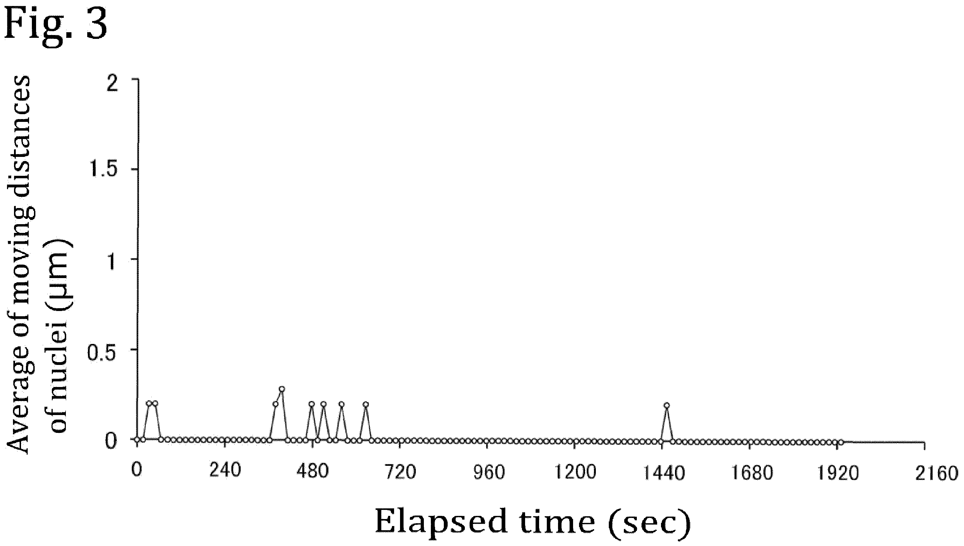

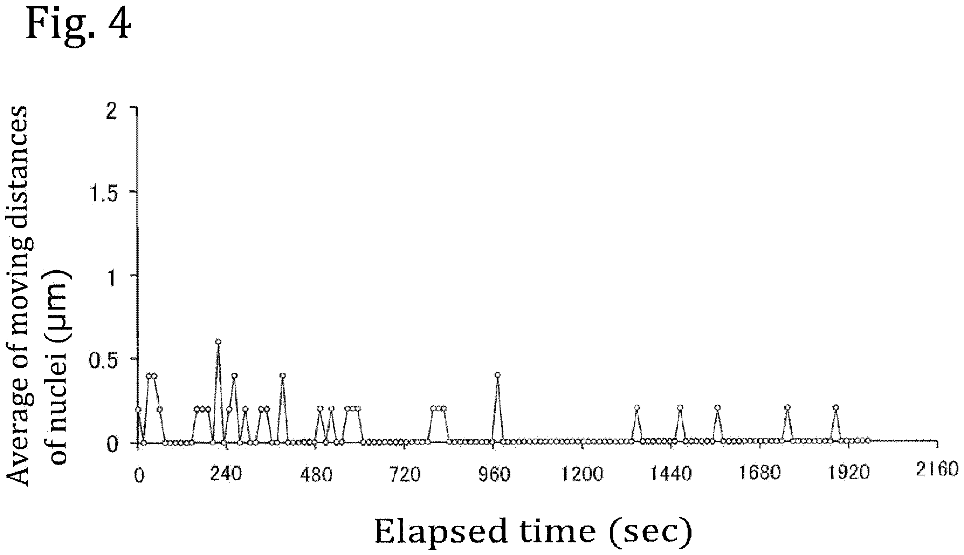

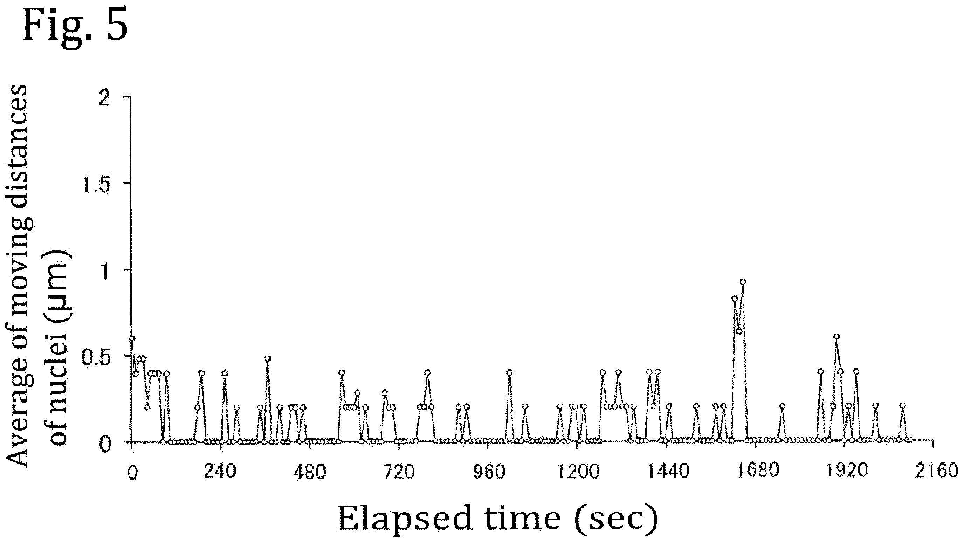

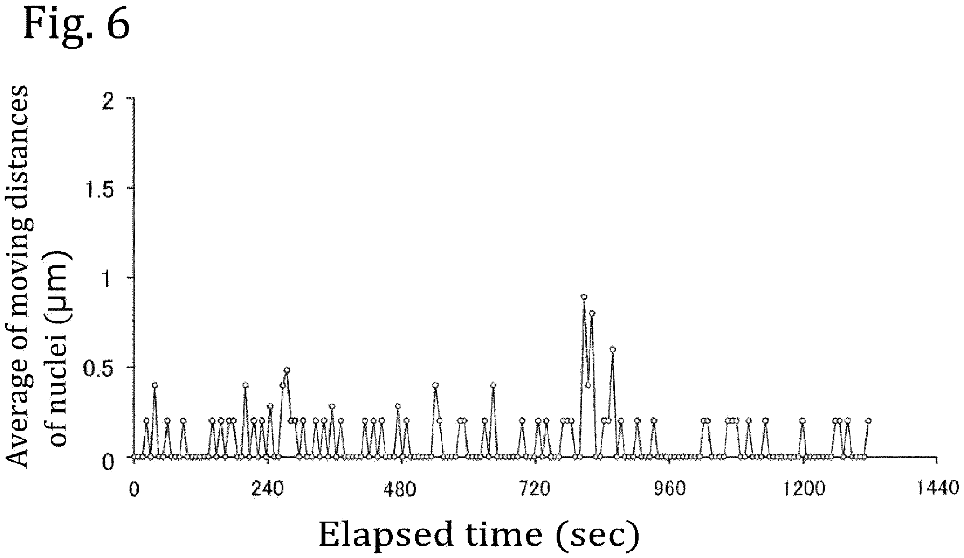

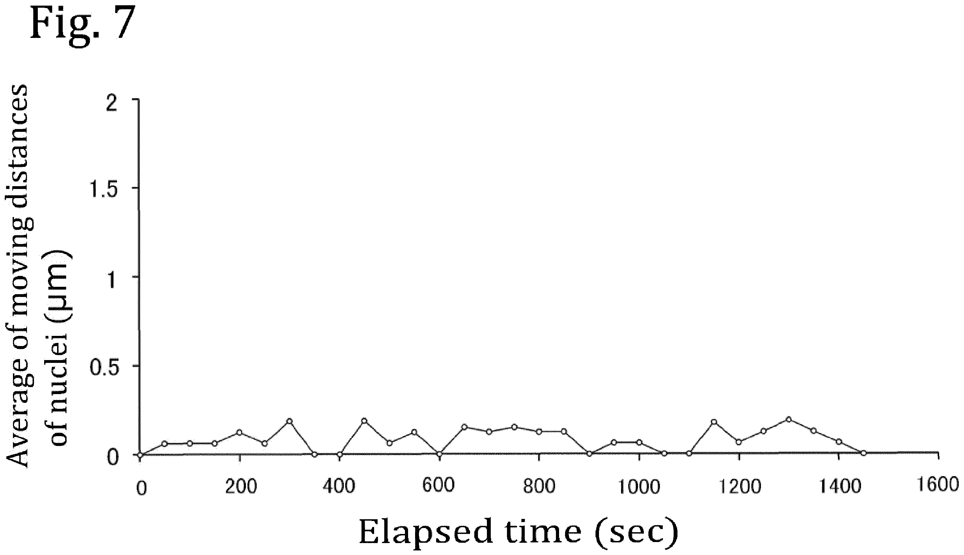

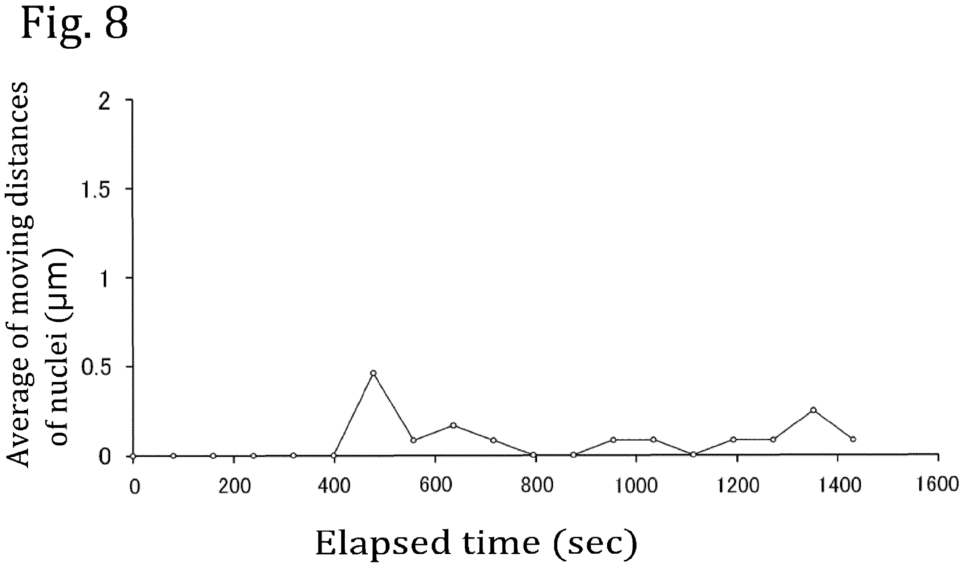

In Test Example 2, results for examining changes of the average of moving distances of nuclei of cells of the secretory duct of the sweat gland after the antiperspirant obtained in Example 2 was contacted with the whole sweat glands are shown in Fig. 3; results for examining changes of the average of moving distances of nuclei of cells of the secretory duct of the sweat gland after the antiperspirant obtained in Example 3 was contacted with the whole sweat glands are shown in Fig. 4; results for examining changes of the average of moving distances of nuclei of cells of the secretory duct of the sweat gland after the antiperspirant obtained in Example 4 was contacted with the whole sweat glands are shown in Fig. 5; results for examining changes of the average of moving distances of nuclei of cells of the secretory duct of the sweat gland after the antiperspirant obtained in Example 5 was contacted with the whole sweat glands are shown in Fig. 6; results for examining changes of the average of moving distances of nuclei of cells of the secretory duct of the sweat gland after the antiperspirant obtained in Example 6 was contacted with the whole sweat glands are shown in Fig. 7; and results for examining changes of the average of moving distances of nuclei of cells of the secretory duct of the sweat gland after the antiperspirant obtained in Example 7 was contacted with the whole sweat glands are shown in Fig. 8. In addition, the term of "Average of moving distances of nuclei" shown in each of the figures means the average of moving distances of nuclei of cells of the secretory duct of the sweat gland.

Whether or not nuclei of cells of the secretory duct of the sweat gland moved was determined based on the results shown in Fig. 3 to Fig. 8, and evaluated in accordance with the following criteria for evaluation.

### «Criteria for evaluation»

The maximum value of the average of moving distances of nuclei of cells of the secretory duct of the sweat gland is 1 µm or less: the nuclei of cells of the secretory duct of the sweat gland are not moved.

The maximum value of the average of moving distances of nuclei of cells of the secretory duct of the sweat gland exceeds 1 µm: the nuclei of cells of the secretory duct of the sweat glands are moved.

As a result, when the antiperspirant containing 18α-glycyrrhetinic acid (Example 2), the antiperspirant containing 18β-glycyrrhetinic acid (Example 3), the antiperspirant containing ursolic acid (Example 4), the antiperspirant containing oleanolic acid (Example 5), the antiperspirant containing α-amylin (Example 6) or the antiperspirant containing β-amylin (Example 7) was contacted with the whole sweat glands, the nuclei of cells of the secretory duct of the sweat gland hardly moved. From these results, it can be seen that the antiperspirants obtained in Examples 2 to 7 act on the secretory duct of the sweat gland, and suppress movement of cells of the secretory duct, and thereby movement of the secretory duct is suppressed. Accordingly, it can be seen that the antiperspirants obtained in Examples 2 to 7 can control the movement of the sweat gland, to control the secretion of sweat.

### Test Example 3

The antiperspirant obtained in Example 1 is applied to each armpit of ten expert panelists having the same proficiency in evaluation skill, and then the expert panelists are subjected to a light exercise. Thereafter, each of the expert panelists gives a score of the degree of sweating on the basis of the following criteria for evaluation. In addition, each of the expert panelists gives a score of the degree of sweating in the same manner as in the above except that the control sample of Comparative Example 1 is used in place of the antiperspirant obtained in Example 1. The average of the scores given by the expert panelists is calculated.

### <Criteria for evaluation>

0 point: sweat in a large amount.
1 point: usual sweat.
2 points: slightly sweat.
3 points: little sweat.
4 points: no sweat.

As a result, when the antiperspirant obtained in Example 1 is used, it is observed that the average of the scores tends to be higher than the average of the scores of the control sample of Comparative Example 1. Incidentally, when an antiperspirant containing the compound represented by the formula (IX), an antiperspirant containing the compound represented by the formula (X), an antiperspirant containing the compound represented by the formula (I) other than the compounds represented by the formulae (IX) and (X), an antiperspirant containing the derivative of the compound represented by the formula (I), an antiperspirant containing the compound represented by the formula (VII) or an antiperspirant containing the derivative of the compound represented by the formula (VII) is used in place of the antiperspirant obtained in Example 1, it is recognized that each of the antiperspirants shows the same tendency as the antiperspirant obtained in Example 1. Accordingly, it can be seen that the antiperspirant of the present invention controls movement of the sweat gland, to control the secretion of sweat.

As described above, the antiperspirant of the present invention includes the above-mentioned pentacyclic compound as an active ingredient for controlling secretion of sweat from sweat glands, and therefore, the antiperspirant can act on the secretory duct of the sweat gland, to control movement of the secretory duct of the sweat gland in secreting sweat. Thus, the antiperspirant of the present invention can act on the sweat gland without blocking of the sweat pore, to control secretion of sweat. Accordingly, the antiperspirant of the present invention is expected to be used as, for example, an antiperspirant used in cosmetic uses, an antiperspirant used in medical uses and the like. In addition, the antiperspirant of the present invention is expected to be used, for example, as an antiperspirant which responds to a user's demand, such as an antiperspirant for completely inhibiting secretion of sweat or an antiperspirant for alleviating secretion of sweat.

## Claims

1. An antiperspirant for suppressing the secretion of sweat, which comprises as an active ingredient for suppressing the secretion of sweat, a pentacyclic compound represented by the formula (I): 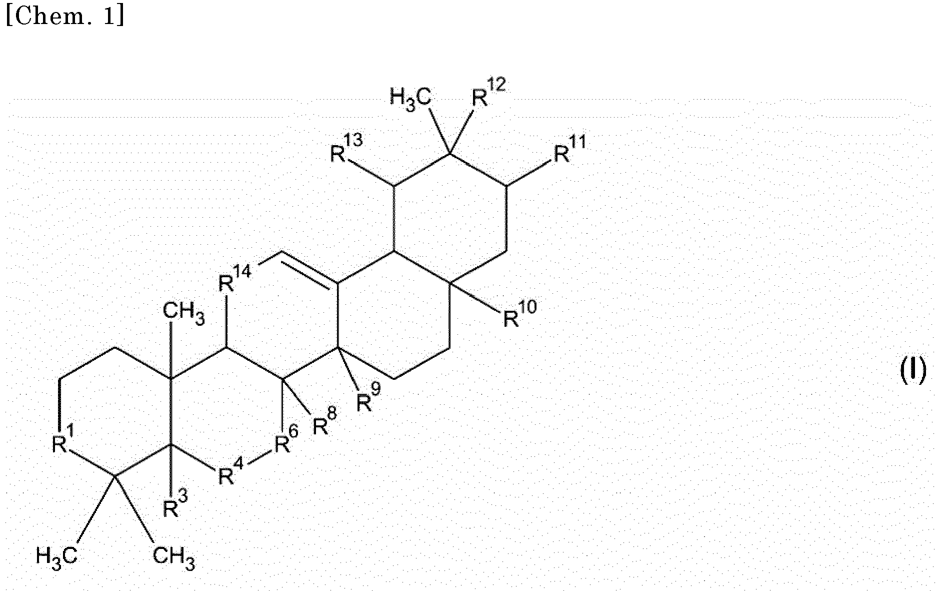 wherein R¹ is a group represented by the formula (II): 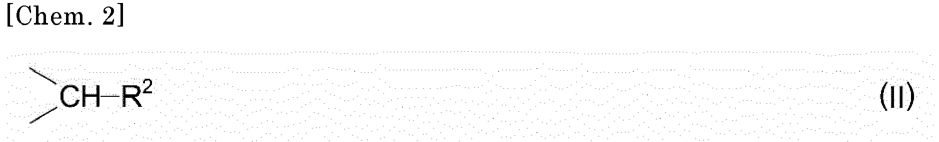 wherein R² is hydrogen atom, hydroxyl group, an alkoxy group having 1 to 12 carbon atoms which may have a substituent, an alkanoyl group having 1 to 12 carbon atoms which may have a substituent, an alkanoyloxy group having 1 to 12 carbon atoms which may have a substituent, or glucopyranosyloxy group which may have a substituent, or
a group represented by the formula (III): 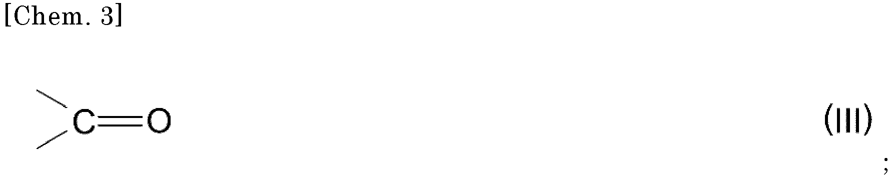 R³ is hydrogen atom or hydroxyl group; R⁴ is the group represented by the formula (III) or a group represented by the formula (IV): 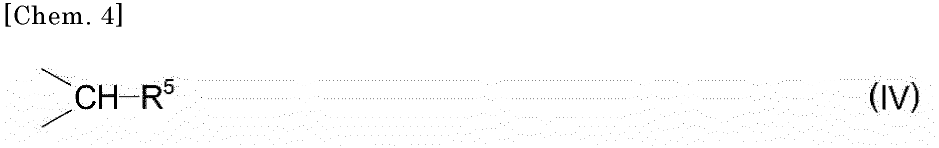 wherein R⁵ is hydrogen atom, a halogen atom, hydroxyl group, or an alkyl group having 1 to 6 carbon atoms;
R⁶ is the group represented by the formula (III) or a group represented by the formula (V): 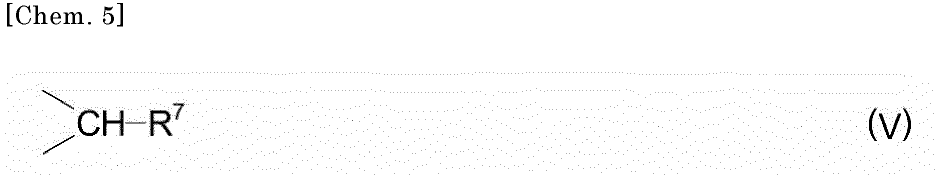 wherein R⁷ is hydrogen atom, hydroxyl group or an alkyl group having 1 to 6 carbon atoms;
each of R⁸ and R⁹ is independently hydrogen atom, hydroxyl group or an alkyl group having 1 to 6 carbon atoms; R¹⁰ is an alkyl group having 1 to 6 carbon atoms or carboxyl group; R¹¹ is hydrogen atom, hydroxyl group, an alkyl group having 1 to 6 carbon atoms or carboxyl group; R¹² is hydrogen atom, an alkyl group having 1 to 6 carbon atoms or carboxyl group; R¹³ is hydrogen atom or an alkyl group having 1 to 6 carbon atoms; and R¹⁴ is the group represented by the formula (III) or a group represented by the formula (VI): 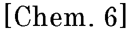 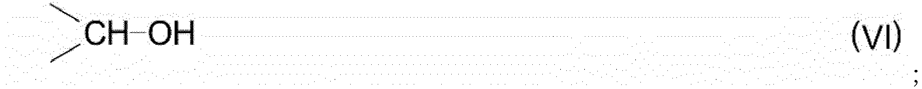 a derivative of the pentacyclic compound represented by the formula (I); a pentacyclic compound represented by the formula (VII): 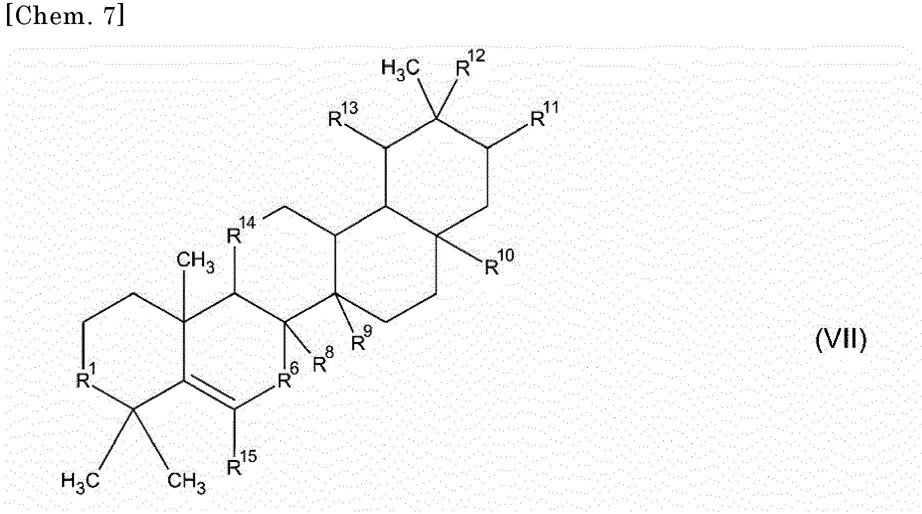 wherein each of R¹, R⁶, R⁸, R⁹, R¹⁰, R¹¹, R¹², R¹³ and R¹⁴ is the same as defined above, and R¹⁵ is hydrogen atom, a halogen atom or hydroxyl group; and
a derivative of the pentacyclic compound represented by the formula (VII).
